# EUROPÄISCHE PATENTSCHRIFT

(11) **EP 0 552 658 B1**
(45) Veröffentlichungstag und Bekanntmachung des Hinweises auf die Patenterteilung: **23.05.2001**
(21) Anmeldenummer: 93100400.6
(22) Anmeldetag: 13.01.1993
(51) Int. Cl.: C07D 239/26, C09K 19/34, C07D 239/34, C07D 285/12, C07D 401/04, C07D 405/12

(54) **Derivate der 3-Cyclohexylpropionsäure und ihre Verwendung in ferroelektrischen Flüssigkristallmischungen**
3-Cyclohexyl propionic acid derivatives and their use in ferroelectric liquid crystal mixtures
Dérivés de l'acide 3-cyclohexyl propionique et leur utilisation dans des mélanges de cristaux liquides ferroélectriques

(30) Priorität: 22.01.1992 DE 4201598
(43) Veröffentlichungstag der Anmeldung: 28.07.1993
(73) Patentinhaber: Aventis Research & Technologies GmbH & Co. KG, 65926 Frankfurt am Main (DE)
(72) Erfinder: Escher, Claus, Dr., W-6109 Mühltal (DE); Illian, Gerhard, Dr., Nerimaku, Tokyo 176 (JP); Schlosser, Hubert, Dr., W-6246 Glashütten/Ts (DE); Wingen, Rainer, Dr., W-6234 Hattersheim am Main (DE)
(74) Vertreter: Bardehle, Heinz, Dipl.-Ing.

(56) Entgegenhaltungen:
- EP-A- 0 104 327
- DD-A- 155 063
- DD-A- 284 893
- GB-A- 2 235 192

## Beschreibung

Flüssigkristalle haben insbesondere im letzten Jahrzehnt Eingang in verschiedene technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen.auf den dielektrischen Ausrichtungseffekten in den nematischen, cholesterischen und/oder smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen zu lang. Dieser Nachteil macht sich insbesondere dann bemerkbar, wenn eine große Anzahl von Bildpunkten angesteuert werden muß. Die Herstellungskosten von Geräten, die größere Bildschirmflächen enthalten, sind dann im allgemeinen zu hoch.

Neben den nematischen und cholesterischen Flüssigkristallen haben seit einigen wenigen Jahren in zunehmendem Maße auch optisch aktive smektische (ferroelektrische) Flüssigkristall-Phasen an Bedeutung gewonnen.

Clark und Lagerwall konnten zeigen, daß die Verwendung ferroelektrischer Flüssigkristallsysteme in sehr dünnen Zellen zu elektrooptischen Schalt- oder Anzeigeelementen führt, die im Vergleich zu den herkömmlichen TN ("twisted nematic")-Zellen um bis zu einem Faktor 1000 schnellere Schaltzeiten aufweisen (vgl. z.B. Lagerwall et al. "Ferroelectric Liquid Crystals for Displays", SID Symposium, October Meeting 1985, San Diego, Cal., USA). Aufgrund dieser und anderer günstiger Eigenschaften, z.B. der bistabilen Schaltmöglichkeit und des nahezu blickwinkelunabhängigen Kontrasts, sind FLCs grundsätzlich für die obengenannten Anwendungsgebiete, z.B. über eine Matrixansteuerung, gut geeignet. Auch auf dem Gebiet der räumlichen Lichtmodulatoren (vgl. z.B. U.Efron in "Spatial Light Modulators and Applications", SPIE vol. 1150, S. 46 ff.) sind ferroelektrische Flüssigkristalle wegen ihres hohen Kontrasts und Geschwindigkeit besonders geeignet. Die Geschwindigkeit ferroelektrischer Flüssigkristallmischungen reicht im allgemeinen aber noch nicht aus, um z.B. hochauflösende, schnelle Anzeigeelemente zu betreiben. Daher ist es wünschenswert, Komponenten aufzufinden, die die Schaltgeschwindigkeit flüssigkristalliner Mischungen steigern. Gegenstand der Erfindung sind deshalb Komponenten, die die Schaltzeit von Flüssigkristallmischungen herabsetzen.

Flüssigkristalline Derivate der 3-Cyclohexylpropionsäure werden in DD 284 893 beschrieben, und zur Verbesserung des Multiplexverhältnisses in elektrooptischen Bauelementen mit nematischen Flüssigkristallmischungen eingesetzt.

GB-A-2 235 192 betrifft Difluorphenylester und deren Verwendung als flüssigkristalline Materialien. Die Säurekomponente weist zwei direkt aneinander gebundene Ringsysteme auf.

DD 155 063 betrifft ein Verfahren zur Herstellung von trans-3-[4-Alkylcyclohexyl]propansäuren und ihren Estern. Die Verbindungen weisen flüssigkristalline Eigenschaften auf.

EP-A-0 104 327 betrifft Ringverbindungen und ihre Verwendung als Komponenten flüssigkristalliner Dielektrika. Bei den Ringverbindungen handelt es sich um Ester, die sowohl im Alkohol- als auch im Esterrest eine zyklische Gruppe aufweisen. Die Verbindungen können als flüssigkristalline Komponenten verwendet werden.

Es wurde nun überraschend gefunden, daß Verbindungen gemäß Formel I sich ausgezeichnet als Mischungskomponenten für ferroelektrische Flüssigkristallmischungen (insbesondere smektisch C*) eignen. Sie sind gut mischbar, besitzen geringere Viskositäten - führen also zu kurzen Schaltzeiten - und fördern die Ausbildung der für eine gute Orientierung im Display vorteilhaften nematischen (cholesterischen) Phase im Temperaturbereich oberhalb der smektischen C*-Phase.

Gegenstand der Erfindung ist daher die Verwendung der Verbindungen gemäß Formel I: 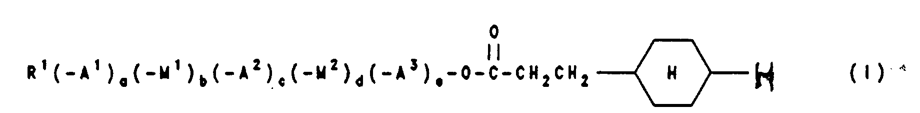 in ferroelektrischen Flüssigkristallmischungen.
In der allgemeinen Formel I haben die Symbole und Indices folgende Bedeutung:
R¹ ist H oder ein geradkettiger oder verzweigter Alkylrest mit 1 bis 22 C-Atomen (mit oder ohne asymmetrisches C-Atom), wobei auch eine oder zwei nicht benachbarte -CH₂-Gruppen durch -O-, -S-, -CO-, -CO-O-, -O-CO-, -CO-S-, -S-CO-, -O-CO-O-, -CH=CH-, -C≡C-, 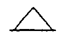 oder -Si(CH₃)₂- ersetzt sein können und auch ein oder mehrere H-Atome des Alkylrestes durch F oder Cl ersetzt sein können, oder eine der nachfolgenden chiralen Gruppen: 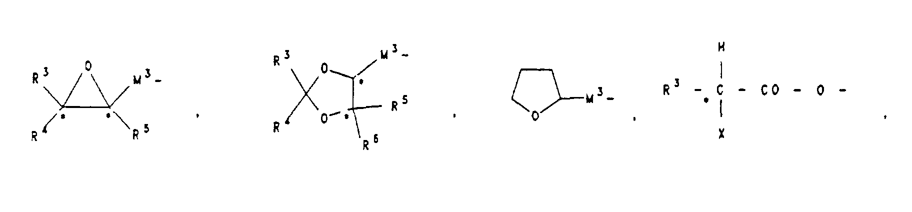
R³, R⁴, R⁵, R⁶ sind unabhängig voneinander H oder ein geradkettiger oder verzweigter Alkylrest mit 1 bis 22 C-Atomen (mit oder ohne asymmetrisches C-Atom), wobei auf eine oder zwei nicht-benachbarte -CH₂-Gruppen durch -O-, -S-,-CO-, -O-CO-, -CO-O-, -CO-S-, -S-CO-, -O-CO-O-, -CH=CH-, -C≡C-, 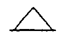 oder -Si(CH₃)₂- ersetzt sein kann und wobei R³ und R⁴ zusammen auch -(CH₂)₄- oder-(CH₂)₅- sein können, wenn sie als Substituenten an ein Dioxolan-System gebunden sind,
X bedeutet -Cl, -Br, -J oder -CN;
A¹, A², A³ sind gleich oder verschieden 1,4-Phenylen, wobei ein oder zwei H-Atome durch F, Cl und/oder CN ersetzt sein können, Pyridazin-3,6-diyl, Pyrazin-2,5-diyl oder Pyridin-2,5-diyl oder Pyrimidin-2,5-diyl, bei denen ein oder zwei H-Atome durch F ersetzt sein können, trans-1,4-Cyclohexylen, bei dem ein oder zwei H-Atome durch -CN und/oder -CH₃ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Naphthalin-2,6-diyl, Bicyclo[2.2.2]octan-1,4-diyl, 1,3-Dioxaborinan-2,5-diyl oder trans-Dekalin-2,6-diyl;
M¹, M² sind gleich oder verschieden, -O-, -S-, -CO-, -CO-O-, -O-CO-, -CO-S-, -S-CO-, -O-CO-O-, -CH₂-O-, -O-CH₂-, -CH₂CH₂-, -CH=CH- oder -C≡C;
M³ ist -CH₂-O-, -CO-O-, -CH₂OC(=O)-;
a, b, c, d, e sind null oder 1, unter der Bedingung, daß die Summe a+c+e 2 oder 3 ist;
* ist ein chirales Zentrum.

Die Herstellung der erfindungsgemäßen Verbindungen kann nach an sich literaturbekannten Methoden erfolgen. 3-Cyclohexylpropionsäuren lassen sich sowohl nach dem von Zaschke et al. in DD 155 063 beschriebenen Verfahren, als auch nach der Methode von Sin et al. (siehe Tetrahedron Letters, Vol. 29, 1988, S. 1759 - 1762) synthetisieren. Je nach Herstellverfahren oder Art des Substituenten R² resultieren 3-Cyclohexylpropionsäuren mit unterschiedlicher Verteilung aus cis-/trans-Isomeren. Die Darstellung der Zielverbindungen der allgemeinen Formel (I) kann dann durch Veresterung dieser 3-Cyclohexylpropionsäuren mit Alkoholen der allgemeinen Formel R¹(-A¹)ₐ(-M¹)_{b}(-A²)_{c}(-M²)_{d}(-A³)ₑ-OH unter Zuhilfenahme geeigneter Kondensationsmittel, z.B. Carbodiimiden (siehe z.B. Houben-Weyl, Methoden der Organischen Chemie, Georg Thieme Verlag, Stuttgart) erfolgen. Dabei können die Verbindungen der Formel (I) ebenfalls als cis-trans-Gemische wechselnder Zusammensetzung anfallen. Durch geeignete Maßnahmen können jedoch auch sowohl auf der Stufe der 3-Cyclohexylpropionsäuren als auch der Verbindungen (I) reine cis- oder trans-Isomere erhalten werden. Sowohl die cis-trans-Gemische von (I) als auch reine cis- bzw. trans-Isomere von (I) lassen sich erfindungsgemäß einsetzen.

Die erfindungsgemäßen Verbindungen der allgemeinen Formel I werden als Komponenten für ferroelektrische Flüssigkristallmischungen eingesetzt. Dabei können die LC-Mischungen 0,1 bis 80 Gew.-%, bevorzugt 0,5 bis 40 Gew.-%, besonders bevorzugt 1,0 bis 20 Gew.-%, enthalten. Die anderen Bestandteile werden vorzugsweise ausgewählt aus den bekannten Verbindungen mit nematischen, cholesterischen und/oder smektischen Phasen; dazu gehören beispielsweise Schiffsche Basen, Biphenyle, Terphenyle, Phenylcyclohexane, Cyclohexylbiphenyle, N-, S- oder O-haltige Heterocyclen, z.B. Pyrimidine, Zimtsäureester, Cholesterinester oder verschieden überbrückte, terminalpolar mehrkernige Ester von p-Alkylbenzoesäuren.

Der Einsatz der erfindungsgemäßen Verbindungen führt zu einer Verbreiterung des Arbeitstemperaturbereiches, indem die SmC*-Phase zu höheren Temperaturen ausgedehnt und der Schmelzpunkt gesenkt wird. Ein besonderer Vorteil ist dabei, daß gleichzeitig die (chiral)nematische Phase N* bewahrt bzw. sogar verbreitert wird, was bessere Orientierungseigenschaften zur Folge hat. Als für die Eignung in elektrooptischen Schaltelementen besonders vorteilhaft hat sich die geringe Rotationsviskosität herausgestellt, die zu geringen Schaltzeiten, oder äquivalent, jedoch für die Displayanwendung von direkter Relevanz, zu geringen Pulsdauern der zum Schalten notwendigen Spannungsimpulse gegebener Amplitude führt.

Die Mischungen können Anwendung finden in elektrooptischen oder vollständig optischen Elementen, z.B. Anzeigeelementen, Schaltelementen, Lichtmodulatoren, Elementen zur Bildbearbeitung, Signalverarbeitung oder allgemein im Bereich der nichtlinearen Optik.

Die Erfindung wird durch die nachfolgenden Beispiele näher erläutert:

Die Phasenumwandlungstemperaturen wurden beim Aufheizen mit Hilfe eines Polarisationsmikroskops anhand der Texturänderungen bestimmt. Die Bestimmung des Schmelzpunkts wurde hingegen mit einem DSC-Gerät durchgeführt. Die Angabe der Phasenumwandlungstemperaturen zwischen den Phasen

| | |
|---|---|
| Nematisch | (N bzw. N*) |
| Smektisch-C | (S_{C} bzw. S_{C}*) |
| Smektisch-A | (S_{A}) |
| Kristallin | (X) |

erfolgt in °C und die Werte stehen zwischen den Phasenbezeichnungen in der Phasenfolge.

### Beispiel 1:

### 3-Cyclohexylpropionsäure-[2-(4-octyloxyphenyl)pyrimidin-5-yl]-ester

3,00 g (10,0 mmol) 5-Hydroxy-2-(4-octyloxyphenyl)-pyrimidin, 1,56 g (10,0 mmol) 3-Cyclohexylpropionsäure, 2,06 g (10,0 mmol) N,N-Dicyclohexylcarbodiimid und 0,1 g 4-N,N-Dimethylaminopyridin in 80 ml Dichlormethan werden 18 Stunden bei Raumtemperatur gerührt. Anschließend wird von unlöslichen Bestandteilen abfiltriert, eingeengt und der Rückstand durch Chromatographie (Kieselgel/Dichlormethan) und Umkristallisation aus Hexan gereinigt. Es werden 2,52 g 3-Cyclohexylpropionsäure-2-(4-octyloxyphenyl)pyrimidin-5-yl-ester erhalten.

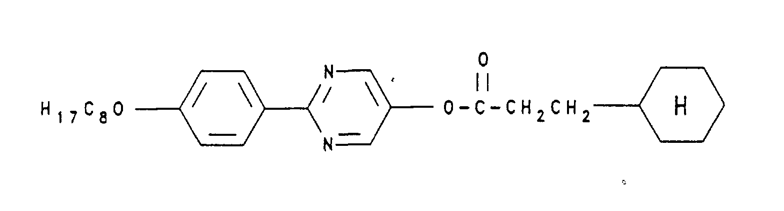

Die Verbindung zeigt die Phasenfolge:
X 66 N 87 I

### Beispiel 2:

### 3-Cyclohexylpropionsäure-4-(5-octyloxypyrimidin-2-yl)phenyl-ester

Die Synthese erfolgte analog Beispiel 1

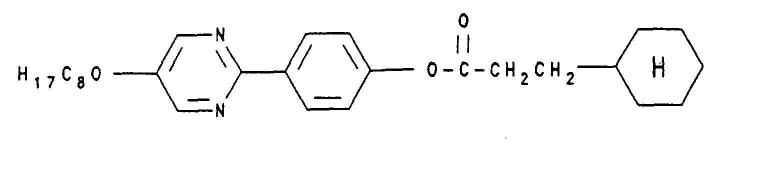

Die Verbindung zeigt die Phasenfolge:
X 97 I

## Patentansprüche

1. Verwendung der Verbindung der allgemeinen Formel I 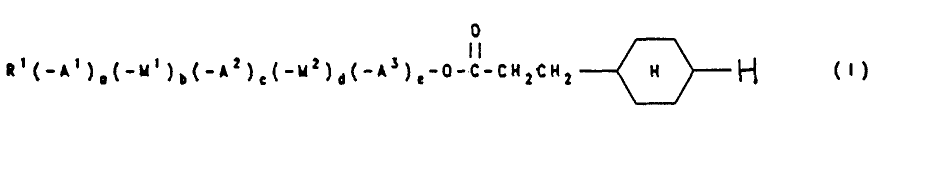 in der die Symbole und Indizes folgende Bedeutung haben:
R¹ ist H oder ein geradkettiger oder verzweigter Alkylrest mit 1 bis 22 C-Atomen (mit oder ohne asymmetrisches C-Atom), wobei auch eine oder zwei nicht benachbarte -CH₂-Gruppen durch -O-, -S-, -CO-, -CO-O-, -O-CO-, -CO-S-, -S-CO-, -O-CO-O-, -CH=CH-, -C≡C-, 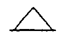 oder -Si(CH₃)₂- ersetzt sein können und auch ein oder mehrere H-Atome des Alkylrestes durch F oder Cl ersetzt sein können, oder eine der nachfolgenden chiralen Gruppen: 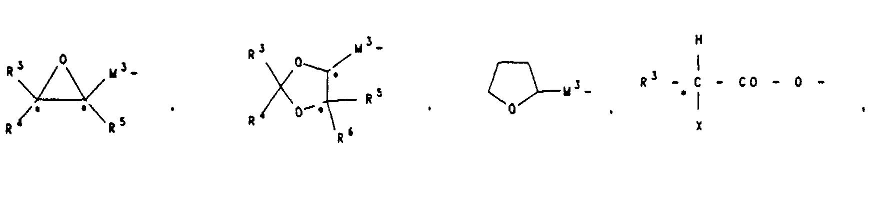
R³, R⁴, R⁵, R⁶ sind unabhängig voneinander H oder ein geradkettiger oder verzweigter Alkylrest mit 1 bis 22 C-Atomen (mit oder ohne asymmetrisches C-Atom), wobei auf eine oder zwei nicht-benachbarte -CH₂-Gruppen durch -O-, -S-, -CO-, -O-CO-, -CO-O-, -CO-S-, -S-CO-, -O-CO-O-, -CH=CH-, -C≡C-, 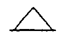 oder -Si(CH₃)₂- ersetzt sein kann und wobei R³ und R⁴ zusammen auch -(CH₂)₄- oder -(CH₂)₅- sein können, wenn sie als Substituenten an ein Dioxolan-System gebunden sind;
X bedeutet -Cl, -Br, -J oder -CN,
A¹, A², A3 sind gleich oder verschieden 1,4-Phenylen, wobei ein oder zwei H-Atome durch F, Cl und/oder CN ersetzt sein können, Pyridazin-3,6-diyl, Pyrazin-2,5-diyl oder Pyridin-2,5-diyl oder Pyrimidin-2,5-diyl, bei dem ein oder zwei H-Atome durch F ersetzt sein können, trans-1,4-Cyclohexylen, bei dem ein oder zwei H-Atome durch -CN und/oder -CH₃ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Naphthalin-2,6-diyl, Bicyclo[2.2.2]-octan-1,4-diyl, 1,3-Dioxaborinan-2,5-diyl oder trans-Dekalin-2,6-diyl;
M¹, M² sind gleich oder verschieden, -O-, -S-, -CO-, -CO-O-, -O-CO-, -CO-S-, -S-CO-, -O-CO-O-, -CH₂-O-, -O-CH₂-, -CH₂CH₂-, -CH=CH- oder -C≡C;
M³ ist -CH₂-O-, -CO-O-, -CH₂OC(=O)-,
a, b, c, d, e sind null oder 1, unter der Bedingung, daß die Summe a+c+e 2 oder 3 ist;
* ist ein chirales Zentrum;
in ferroelektrischen Flüssigkristallmischungen.

2. Verwendung nach Anspruch 1, dadurch gekennzeichnet, daß Verbindungen der Formel I ferroelektrischen Flüssigkristallmischungen in Anteilen von 0,1 bis 80 Gew.-% zugesetzt werden.

3. Ferroelektrische Flüssigkristallmischung, enthaltend eine oder mehrere Verbindungen der Formel (I) wie sie in Anspruch 1 oder 2 definiert sind.

## Claims

1. The use of a compound of the formula I, 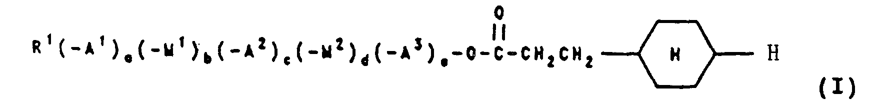 in which the symbols and indices have the following meanings:
R¹ is H or a straight-chain or branched alkyl radical having 1 to 22 carbon atoms (with or without an asymmetric carbon atom) in which, in addition, one or two non-adjacent -CH₂- groups may be replaced by -O-, -S-, -CO-, -CO-O-, -O-CO-, -CO-S-, -S-CO-, -O-CO-O-, -CH=CH-, -C=C-, 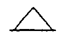 or -Si(CH₃)₂- and in which one or more H atoms of the alkyl radical may be replaced by F or Cl, or is one of the following chiral groups: 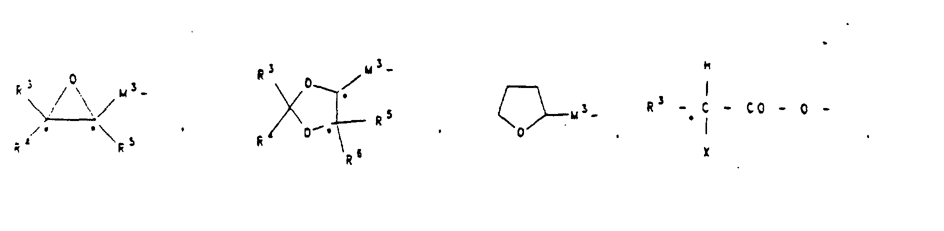
R³, R⁴, R⁵ and R⁶, independently of one another, are H or a straight-chain or branched alkyl radical having 1 to 22 carbon atoms (with or without an asymmetric carbon atom) in which, in addition, one or two non-adjacent -CH₂- groups may be replaced by -O-, -S-, -CO-, -O-CO-, -CO-O-, -CO-S-, -S-CO-, -O-CO-O-, -CH=CH-, -C≡C-, 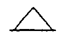 or -Si(CH₃)₂- and in which R³ and R⁴ together may alternatively be -(CH₂)₄- or -(CH₂)₅- if they are bonded, as substituents, to a dioxolane system,
X is -Cl, -Br, -I or -CN,
A¹, A² and A³ are identical or different and are 1,4-phenylene in which one or two H atoms may be replaced by F, Cl and/or CN, pyridazine-3,6-diyl, pyrazine-2,5-diyl, pyridine-2,5-diyl or pyrimidine-2,5-diyl in which one or two H atoms may be replaced by F, trans-1,4-cyclohexylene in which one or two H atoms may be replaced by -CN and/or -CH₃, 1,3,4-thiadiazole-2,5-diyl, 1,3-dioxane-2,5-diyl, 1,3-dithiane-2,5-diyl, 1,3-thiazole-2,4-diyl, 1,3-thi-azole-2,5-diyl, thiophene-2,4-diyl, thiophene-2,5-diyl, piperazine-1,4-diyl, piperazine-2,5-diyl, naphthalene-2,6-diyl, bicyclo[2.2.2]octane-1,4-diyl, 1,3-dioxaborinane-2,5-diyl or trans-dekalin-2,6-diyl,
M¹ and M² are identical or different and are -O-, -S-, -CO-, -CO-O-, -O-CO-, -CO-S-, -S-CO-, -O-CO-O-, -CH₂-O-, -O-CH₂-, -CH₂CH₂-, -CH=CH- or -C≡C,
M³ is -CH₂-O-, -CO-O-, or -CH₂-OC(=O)-,
a,b,c,d and e are zero or 1, with the proviso that the sum a+c+e is 2 or 3,
* is a chiral center,
in ferroelectric liquid-crystal mixtures.

2. The use as claimed in claim 1, wherein a compound of the formula I is added to a ferroelectric liquid-crystal mixture in an amount of 0.1 to 80 % by weight.

3. A ferroelectric liquid crystal mixture, containing one or more compounds of the formula (I) as defined in claim 1 or 2.

## Revendications

1. Utilisation du composé de formule générale I 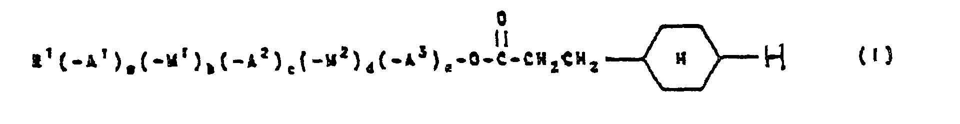 dans laquelle les symboles et indices ont les significations suivantes :
R¹ est H ou un groupe alkyle linéaire ou ramifié avec 1 à 22 atomes de C (avec ou sans atome de C asymétrique), dans lequel aussi un ou deux groupes -CH₂- non adjacents peuvent être remplacés par -O-, -S-, -CO-, -CO-O-, -O-CO-, -CO-S-, -S-CO-, -O-CO-O-, -CH=CH-, -C≡C-, 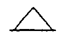 ou -Si(CH₃)₂- et aussi un ou plusieurs atomes de H du groupe alkyle peuvent être remplacés par F ou Cl, ou est l'un des groupes chiraux suivants : 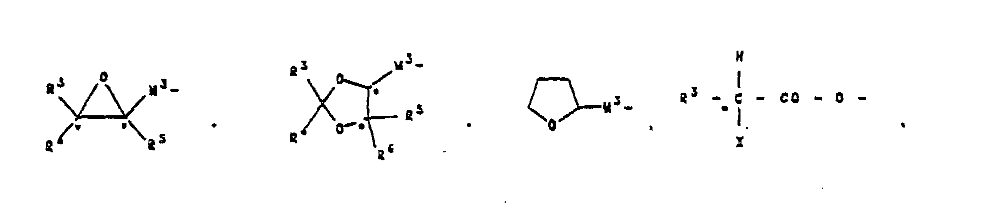
R³, R⁴, R⁵, R⁶, indépendamment les uns des autres, représentent H ou un groupe alkyle linéaire ou ramifié avec 1 à 22 atomes de C (avec ou sans atome de C asymétrique), dans lequel aussi un ou deux groupes -CH₂- non adjacents peuvent être remplacés par -O-, -S-, -CO-, -O-CO-, -CO-O-, -CO-S, -S-CO-, -O-CO-O-, -CH=CH-, -C≡C-, 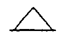 ou -Si(CH₃)₂- et R³ et R⁴ conjointement peuvent être aussi -(CH₂)₄- ou -(CH₂)₅-, quand ils sont liés comme substituants à un système dioxolane ;
X représente : -Cl, -Br, -I ou -CN,
A¹, A², A³, identiques ou différents, représentent : 1,4 phénylène, dans lequel un ou deux atomes de H peuvent être remplacés par F, Cl et/ou CN ; pyridazine-3,6-diyle ; pyrazine-2,5-diyle ou pyridine-2,5-diyle ou pyrimidine-2,5-diyle dans lequel un ou deux atomes de H peuvent être remplacés par F ; trans-1,4-cyclohexylène, dans lequel un ou deux atomes de H peuvent être remplacés par -CN et/ou -CH₃ ; (1,3,4)-thiadiazole-2,5-diyle; 1,3-dioxanne-2,5-diyle; 1,3-dithiane-2,5-diyle ; 1,3-thiazole-2,4-diyle; 1,3-thiazole-2,5-diyle ; thiophène-2,4-diyle ; thiophène-2,5-diyle ; pipérazine-1,4-diyle; pipérazine-2,5-diyle ; naphtalène-2,6-diyle ; bicyclo[2,2,2]-octane-1,4-diyle ; 1,3-dioxaborinane-2,5-diyle ou trans-décaline-2,6-diyle ;
M¹, M², identiques ou différents, représentent : -O-, -S-, -CO-, -CO-O-, -O-CO-, -CO-S-, -S-CO-, -O-CO-O-, -CH₂-O-, -O-CH₂-, -CH₂CH₂-, -CH=CH- ou -C≡C- ;
M³ représente : -CH₂-O-, -CO-O-, -CH₂OC(=O)-,
a, b, c, d, e ont pour valeur 0 ou 1, à condition que la somme a+c+e soit égale à 2 ou 3 ;
* représente un centre chiral ;
dans des mélanges de cristaux liquides ferroélectriques.

2. Utilisation selon la revendication 1, caractérisée en ce que des composés de formule I sont ajoutés à des mélanges de cristaux liquides ferroélectriques en des proportions de 0,1 à 80 % en poids.

3. Mélange de cristaux liquides ferroélectrique contenant un ou plusieurs composés de formule I tels que définis dans la revendication 1 ou 2.
